(19) Europäisches Patentamt
European Patent Office
Office européen des brevets

(11) EP 3 212 158 B1

(12) FASCICULE DE BREVET EUROPEEN

(45) Date de publication et mention de la délivrance du brevet:
**28.11.2018 Bulletin 2018/48**

(21) Numéro de dépôt: **15786983.5**

(22) Date de dépôt: **29.10.2015**

(51) Int Cl.:
***A61K 8/58*** (2006.01) ***A61K 8/81*** (2006.01)
***A61Q 19/08*** (2006.01) ***C08F 220/18*** (2006.01)
***C08F 222/06*** (2006.01) ***C08F 8/30*** (2006.01)

(86) Numéro de dépôt international:
**PCT/EP2015/075124**

(87) Numéro de publication internationale:
**WO 2016/066747 (06.05.2016 Gazette 2016/18)**

# (54) POLYMÈRE ACRYLIQUE À GROUPEMENTS ALCOXYSILANE ET SES APPLICATIONS EN COSMÉTIQUE

ACRYLPOLYMER MIT ALKOXYSILANGRUPPEN UND KOSMETISCHE VERWENDUNGEN DAVON

ACRYLIC POLYMER COMPRISING ALKOXYSILANE GROUPS AND COSMETIC USES THEREOF

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **31.10.2014 FR 1460490**

(43) Date de publication de la demande:
**06.09.2017 Bulletin 2017/36**

(73) Titulaire: **L'OREAL**
**75008 Paris (FR)**

(72) Inventeurs:
- **SCHULTZE, Xavier**
  **F-93320 Les Pavillons sous Bois (FR)**
- **HERNANDEZ, Franck**
  **F-93250 Villemomble (FR)**
- **LION, Bertrand**
  **93601 Aulnay-Sous-Bois (FR)**
- **PORTAL, Julien**
  **F-93320 Les Pavillons sous Bois (FR)**

(74) Mandataire: **Kromer, Christophe**
**L'Oréal**
**Service DIPI**
**9 Rue Pierre Dreyfus**
**92110 Clichy (FR)**

(56) Documents cités:
**WO-A1-98/29092 WO-A1-2012/089798**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 3 212 158 B1

## Description

La présente invention concerne un polymère acrylique à groupements alcoxysilane, une composition comprenant un tel polymère et l'utilisation de ce polymère dans le domaine cosmétique.

Au cours du processus de vieillissement, il apparaît différents signes sur la peau, très caractéristiques de ce vieillissement, se traduisant notamment par une modification de la structure et des fonctions cutanées. Les principaux signes cliniques du vieillissement cutané sont notamment l'apparition de ridules et de rides profondes, en augmentation avec l'âge.

Il est connu de traiter ces signes du vieillissement en utilisant des compositions cosmétiques ou dermatologiques contenant des actifs capables de lutter contre le vieillissement, tels que les $\alpha$-hydroxy-acides, les $\beta$-hydroxy-acides et les rétinoïdes. Ces actifs agissent sur les rides en éliminant les cellules mortes de la peau et en accélérant le processus de renouvellement cellulaire. Toutefois, ces actifs présentent l'inconvénient de n'être efficaces pour le traitement des rides qu'après un certain temps d'application. Or, on cherche de plus en plus à obtenir un effet immédiat des actifs utilisés, conduisant rapidement à un lissage des rides et ridules et à la disparition des marques de fatigue.

Les inventeurs ont découvert qu'un polymère acrylique d'anhydride maléique particulier associé avec un aminosilane particulier présente de bonnes propriétés filmogènes. Lorsque le polymère associé avec ledit aminosilane est appliqué sur la peau, il présente un bon effet tenseur sur la peau et permet ainsi d'atténuer les rides de la peau, en particulier rapidement ou de façon immédiate après l'application sur la peau. Cet effet tenseur présente également une bonne résistance à l'eau et donc une bonne rémanence à l'eau. Ce polymère acrylique particulier est facilement véhiculable dans une huile hydrocarbonée comme l'isododécane. Ce polymère, associé avec ledit aminosilane, est donc bien adapté pour réaliser des compositions anhydres ayant une bonne résistance à l'eau et ayant notamment un bon effet tenseur.

Ce polymère acrylique d'anhydride maléique associé avec ledit aminosilane forme un dépôt filmogène convenant également pour le maquillage de la peau ou des lèvres ou des cils tels que les fonds de teint, les rouges à lèvres, les mascaras, ou bien encore pour la fixation des cheveux.
Le dépôt filmogène obtenu présente une bonne résistance à l'eau. Il présente également une bonne résistance à l'huile (notamment à l'huile d'olive), et au sébum.

De façon plus précise, la présente invention a pour objet un procédé, notamment cosmétique, de soin ou de maquillage des matières kératiniques, en particulier de la peau, plus particulièrement de la peau du visage, en particulier de la peau ridée, comprenant :

soit l'application topique sur les matières kératiniques, en particulier sur la peau, d'un mélange anhydre (extemporané) d'une composition cosmétique comprenant un polymère acrylique d'anhydride maléique et d'un composé amino alcoxysilane (I), ou d'une composition cosmétique le contenant ;
soit l'application séquentielle sur les matières kératiniques, en particulier sur la peau d'une composition cosmétique anhydre comprenant un polymère acrylique d'anhydride maléique et d'un composé amino alcoxysilane (I), ou d'une composition cosmétique anhydre le contenant,
ledit polymère acrylique d'anhydride maléique étant susceptible d'être obtenu par polymérisation de :

(a) 50 à 90 % en poids, du poids total de monomères, de (méth)acrylate d'isobornyle
(b) 1 à 50 % en poids d'anhydride maléique
(c) 0 à 49 % en poids de monomère (méth)acrylate additionnel choisi parmi :

(i) les (métha)crylate d'alkyle en $C_1$-$C_{20}$, linéaire ou ramifié, saturé ou insaturé, éventuellement interrompu par un ou plusieurs hétéroatomes non adjacents choisi parmi O ou S ou par un groupement NR , R étant un groupe alkyle en $C_1$-$C_4$, éventuellement substitué par un groupement phényle ou furfuryle ;
(ii) les (méth)acrylate de cycloalkyle en $C_4$-$C_8$ saturé éventuellement interrompu par O ou NH ;

ledit amino alcoxy silane étant de formule (I) :

$$R\text{-}NH\text{-}R_1Si(OR_2)_z(R_3)_x \qquad (I)$$

dans laquelle :

$R_1$ est un groupe divalent hydrocarbonée en $C_1$-$C_{20}$, linéaire ou ramifiée, saturée ou insaturée, cyclique ou acyclique, pouvant être interrompu dans sa chaine par un hétéroatome (O, S, NH) ou un groupement carbonyle (CO), $R_1$ étant lié à l'atome de silicium directement via un atome de carbone ;
R= H ou groupe alkyle en $C_1$-$C_4$, de préférence H ;

$R_2$ et $R_3$ identiques ou différents, représentent un groupe alkyle, linéaire ou ramifié, comprenant de 1 à 6 atomes de carbone,
z désigne un nombre entier allant de 1 à 3, et
x désigne un nombre entier allant de 0 à 2,
avec z+x =3.

Le procédé selon l'invention convient pour le soin ou le maquillage des matières kératiniques comme la peau, les lèvres, les cils, les cheveux, les ongles.

Le procédé selon l'invention est notamment destiné à être réalisé pour le soin de la peau, plus particulièrement de la peau du visage, en particulier de la peau ridée.

Le procédé selon l'invention est en particulier destiné à lisser la peau humaine du visage et/ou du corps et/ou à diminuer ou effacer les signes du vieillissement cutané, en particulier à réduire ou effacer les rides et/ou les ridules de la peau.

Selon un mode de réalisation du procédé selon l'invention, on effectue l'application topique sur les matières kératiniques d'un mélange extemporané d'une composition anhydre comprenant un polymère acrylique d'anhydride maléique et d'un amino alcoxysilane (I), ou d'une composition anhydre le contenant et comprenant un milieu physiologiquement acceptable, tel que défini ci-après.

Selon un mode de réalisation du procédé selon l'invention, on effectue l'application topique sur la peau d'un mélange extemporané d'une composition anhydre comprenant un polymère acrylique d'anhydride maléique et d'un amino alcoxysilane (I), ou d'une composition anhydre le contenant et comprenant un milieu physiologiquement acceptable, tel que défini ci-après.

Selon un autre mode de réalisation du procédé selon l'invention, on effectue l'application séquentielle sur les matières kératiniques d'une composition anhydre comprenant un polymère acrylique telle que décrite précédemment et d'un amino alcoxysilane (I), ou d'une composition anhydre le contenant et comprenant un milieu physiologiquement acceptable, tels que définis précédemment.

Selon un autre mode de réalisation du procédé selon l'invention, on effectue l'application séquentielle sur la peau d'une composition anhydre comprenant un polymère acrylique telle que décrite précédemment et d'un amino alcoxysilane (I), ou d'une composition anhydre le contenant et comprenant un milieu physiologiquement acceptable, tels que définis précédemment.

L'invention a également pour objet l'utilisation cosmétique en tant qu'agent tenseur de la peau, en particulier d'une peau ridée, d'un polymère acrylique d'anhydride maléique tel que décrit précédemment, en mélange avec un amino alcoxysilane (I), ou d'une composition anhydre le contenant et comprenant un milieu physiologiquement acceptable, tel que défini ci-après.

L'invention a aussi pour objet une composition, notamment cosmétique, obtenue par mélange d'une composition anhydre comprenant ledit polymère acrylique d'anhydride maléique et d'un amino alcoxyosilane (I) ou d'une composition anhydre le contenant et comprenant un milieu physiologiquement acceptable, tel que défini ci-après.

L'invention a encore pour objet un kit comprenant une première composition anhydre comprenant ledit polymère acrylique d'anhydride maléique tel que décrit précédemment et une deuxième composition anhydre comprenant un amino alcoxysilane (I) tel que décrit précédemment et comprenant un milieu physiologiquement acceptable, les première et deuxième compositions étant conditionnées chacune dans un ensemble de conditionnement distinct.
L'ensemble de conditionnement des compositions est de façon connue tout packaging adapté pour stocker les compositions cosmétiques (flacons, tube, flacon spray, flacon aérosol notamment).
Un tel kit permet de mettre en oeuvre le procédé de traitement de la peau selon l'invention.

On entend par « agent tenseur » des composés susceptibles d'avoir un effet tenseur apparent, c'est-à-dire de lisser la peau et réduire, voire faire disparaître, de façon immédiate les rides et les ridules.

L'effet tenseur peut être caractérisé par un test *in vitro* de rétractation tel que décrit à l'exemple 5.

Le polymère acrylique d'anhydride maléique utilisé selon l'invention comprend un (méth)acrylate d'isobornyle, de l'anhydride maléique et éventuellement un monomère acrylate additionnel tels que définis précédemment. Avantageusement, le polymère acrylique d'anhydride maléique est constitué essentiellement de ces monomères selon les teneurs décrites précédemment.

Avantageusement, le polymère utilisé selon l'invention est issu de la polymérisation de :

(a) 50 à 90 % en poids, du poids total de monomères, de (méth)acrylate d'isobornyle
(b) 5 à 50 % en poids d'anhydride maléique.
(c) 0 à 30 % en poids de monomère (méth)acrylate additionnel tel que décrit précédemment.

Avantageusement, le polymère utilisé selon l'invention est issu de la polymérisation de :

(a) 50 à 80 % en poids, du poids total de monomères, de (méth)acrylate d'isobornyle
(b) 5 à 30 % en poids d'anhydride maléique.
(c) 15 à 30 % en poids de monomère (méth)acrylate additionnel tel que décrit précédemment.

Avantageusement, le polymère utilisé selon l'invention est issu de la polymérisation de :

(a) 50 à 80 % en poids, du poids total de monomères, de (méth)acrylate d'isobornyle
(b) 5 à 25 % en poids d'anhydride maléique.
(c) 15 à 30 % en poids de monomère (méth)acrylate additionnel tel que décrit précédemment.

Avantageusement, le polymère utilisé selon l'invention est issu de la polymérisation de :

(a) 50 à 80 % en poids, du poids total de monomères, de (méth)acrylate d'isobornyle
(b) 5 à 15 % en poids d'anhydride maléique.
(c) 15 à 30 % en poids de monomère (méth)acrylate additionnel tel que décrit précédemment.

Avantageusement, le polymère utilisé selon l'invention est issu de la polymérisation de :

(a) 60 à 80 % en poids, du poids total de monomères, de (méth)acrylate d'isobornyle
(b) 5 à 12 % en poids d'anhydride maléique.
(c) 15 à 30 % en poids de monomère (méth)acrylate additionnel tel que décrit précédemment.

Avantageusement, le polymère utilisé selon l'invention est issu de la polymérisation de :

(a) 60 à 80 % en poids, du poids total de monomères, de (méth)acrylate d'isobornyle
(b) 5 à 12 % en poids d'anhydride maléique.
(c) 18 à 30 % en poids de monomère (méth)acrylate additionnel tel que décrit précédemment.

Le monomère (méth)acrylate additionnel est de préférence choisi parmi les (méth)acrylate d'alkyle en $C_6$-$C_{16}$, et préférentiellement choisi parmi les acrylate d'alkyle en $C_6$-$C_{16}$.
Comme exemple de (méth)acrylate d'alkyle en $C_6$-$C_{16}$, on peut citer le (méth)acrylate d'hexyle, le (méth)acrylate d'octyle, le (méth)acrylate de 2-éthyl hexyle, le (méth)acrylate de décyle, le (méth)acrylate de lauryle. On utilise de préférence l'acrylate de 2-éthyl hexyle.
De préférence, le polymère utilisé selon l'invention comprend, ou consiste en, de l'acrylate d'isobornyle, l'acrylate de 2-éthylhexyle et l'anhydride maléique.
Un polymère particulièrement préféré utilisé selon l'invention est issu de la polymérisation de :

(a) 50 à 80 % en poids, du poids total de monomères, de (méth)acrylate d'isobornyle
(b) 5 à 30 % en poids d'anhydride maléique.
(c) 15 à 30 % en poids de monomère acrylate d'alkyle en $C_6$-$C_{16}$.

Un polymère particulièrement préféré utilisé selon l'invention est issu de la polymérisation de :

(a) 50 à 80 % en poids, du poids total de monomères, de (méth)acrylate d'isobornyle
(b) 5 à 25 % en poids d'anhydride maléique.
(c) 15 à 30 % en poids de monomère acrylate d'alkyle en $C_6$-$C_{16}$.

Un polymère particulièrement préféré utilisé selon l'invention est issu de la polymérisation de :

(a) 50 à 80 % en poids, du poids total de monomères, de (méth)acrylate d'isobornyle
(b) 5 à 15 % en poids d'anhydride maléique.
(c) 15 à 30 % en poids de monomère acrylate d'alkyle en $C_6$-$C_{16}$.

Un polymère particulièrement préféré utilisé selon l'invention est issu de la polymérisation de :

(a) 60 à 80 % en poids, du poids total de monomères, de (méth)acrylate d'isobornyle
(b) 5 à 12 % en poids d'anhydride maléique.
(c) 15 à 30 % en poids de monomère acrylate d'alkyle en $C_6$-$C_{16}$.

Un polymère particulièrement préféré utilisé selon l'invention est issu de la polymérisation de :

(a) 60 à 80 % en poids, du poids total de monomères, de (méth)acrylate d'isobornyle
(b) 5 à 12 % en poids d'anhydride maléique.
(c) 18 à 30 % en poids de monomère acrylate d'alkyle en $C_6$-$C_{16}$.

Avantageusement, le polymère utilisé selon l'invention consiste en les monomères décrits précédemment.
Avantageusement, le polymère utilisé selon l'invention est non ionique.
De préférence, le polymère acrylique d'anhydride maléique utilisé selon l'invention a un poids moléculaire moyen en poids allant de 5000 à 1 000 000 g/mole de préférence allant de 10000 à 500 000 g/mole, et préférentiellement allant de 15000 à 350 000 g/mole.
Le poids moléculaire peut notamment être déterminé par chromatogaphie par exclusion stérique , éluant THF, étalon polystyrène, détecteur refractomètre 2414 de chez WATERS.
Le copolymère peut être un polymère statistique, alterné (bloc), à gradient. De préférence, le copolymère est statistique.
Le copolymère utilisé selon l'invention peut être préparé par polymérisation radicalaire des monomères décrits précédemment, notamment en mélange ou ajoutés séquentiellement pendant la polymérisation, notamment en utilisant un solvant organique ayant un point d'ébullition supérieur ou égal à 60 °C, comme par exemple l'isododécane, l'éthanol, l'acétate d'éthyle, le tétrahydrofurane, le méthyltétrahydrofurane, la méthyl éthyl cétone. Le solvant organique permet de solubiliser les monomères mis en oeuvre et le polymère formé.
La polymérisation est notamment effectuée en présence d'un amorceur radicalaire notamment de type peroxyde (par exemple tert-Butyl peroxy-2-ethylhexanoate : Trigonox 21S ; 2,5-dimethyl-2,5-di(2-ethylhexanoylperoxy)hexane :Trigonox 141 ; tert-butyl peroxypivalate : Trigonox 25C75 de chez AkzoNobel) ou azoïque par exemple (AIBN : azobisisobutyronitrile ; V50 : 2,2'-azo-bis(2-amidinopropane) dihydrochlorure).
La polymérisation peut s'effectuer à une température allant de 60 à 100 °C, de préférence allant de 60 à 85 °C. La durée de la polymérisation peut être d'environ 24 heures.
Le polymère utilisé selon l'invention peut être utilisé dans une composition anhydre comprenant un milieu physiologiquement acceptable, en particulier dans une composition cosmétique.
Par milieu physiologiquement acceptable, on entend un milieu compatible avec les matières kératiniques d'êtres humains, en particulier avec la peau.
Par composition cosmétique, on entend une composition compatible avec les matières kératiniques, qui présente une couleur, une odeur et un toucher agréables, et qui ne génère pas d'inconforts inacceptables (picotements, tiraillements, rougeurs), susceptibles d'en détourner le consommateur.
Le polymère acrylique d'anhydride maléique tel que défini précédemment peut être présent dans la composition selon l'invention en une teneur allant de 0,1 à 10 % en poids, par rapport au poids total de la composition, de préférence de 0,5 % à 10 % en poids de matière active, et préférentiellement allant de 1 % à 8 % en poids, et plus préférentiellement allant de 1 % à 6 % en poids.
Pour l'amino alcoxysilane de formule (I) mis en oeuvre dans le procédé selon l'invention: De préférence, $R_2$ représente un groupe alkyle comprenant de 1 à 4 atomes de carbone. De préférence, $R_2$ représente un groupe alkyle linéaire comprenant de 1 à 4 atomes de carbone.
De préférence, $R_2$ représente le groupe éthyle.
De préférence, $R_3$ représente un groupe alkyle comprenant de 1 à 4 atomes de carbone. De préférence, $R_3$ représente un groupe alkyle linéaire comprenant de 1 à 4 atomes de carbone.
De préférence, $R_3$ représente le groupe méthyle ou éthyle.
De préférence, R = H.
De préférence $R_1$ est une chaîne acyclique.
De préférence $R_1$ est une chaîne hydrocarbonée en $C_1$-$C_6$, linéaire ou ramifiée, saturée ou insaturée. Préférentiellement, $R_1$ est une chaîne hydrocarbonée en $C_1$-$C_6$, linéaire saturée. Plus préférentiellement, $R_1$ est une chaîne hydrocarbonée en $C_2$-$C_4$, linéaire saturée.
De préférence, $R_1$ est une chaîne hydrocarbonée en $C_1$-$C_6$, linéaire saturée substituée,

$$R = H ,$$

$R_2$ représente un groupe alkyle comprenant de 1 à 4 atomes de carbone,
$R_3$ représente un groupe alkyle comprenant de 1 à 4 atomes de carbone.
De préférence z est égal à 3.
De préférence, l'amino alcoxysilane de formule (I) est choisi parmi le 3-aminopropyltriéthoxysilane (APTES),

le 3-aminoéthyltriéthoxysilane (AETES), le 3-aminopropylméthyldiéthoxysilane, le N-(2-aminoéthyl)-3-aminopropyltriéthoxysilane, le 3-(m-aminophénoxy)propyltriméthoxysilane, le p-aminophényltriméthoxysilane, le N-(2-aminoéthylaminométhyl)phénéthyltriméthoxysilane.
De préférence, l'amino alcoxysilane (I) est choisi parmi le 3-aminopropyltriéthoxysilane (APTES), le 3-aminoéthyltriéthoxysilane (AETES), le 3-aminopropylméthyldiéthoxysilane, le N-(2-aminoéthyl)-3-aminopropyltriéthoxysilane.
De préférence l'amino alcoxysilane (I) est le 3-aminopropyl triéthoxysilane (APTES).

Avantageusement, l'amino alcoxysilane est mis en oeuvre selon un ratio molaire amino alcoxysilane / groupe anhydride maléique du polymère acrylique allant de 0,01 à 10, de préférence allant de 0,1 à 5, préférentiellement allant de 0,1 à 2, et plus préférentiellement allant de 0,1 à 1.

L'amino alcoxysilane (I) mis en oeuvre réagit avec le groupe anhydride maléique présent dans le polymère pour former une unité de formule suivante :

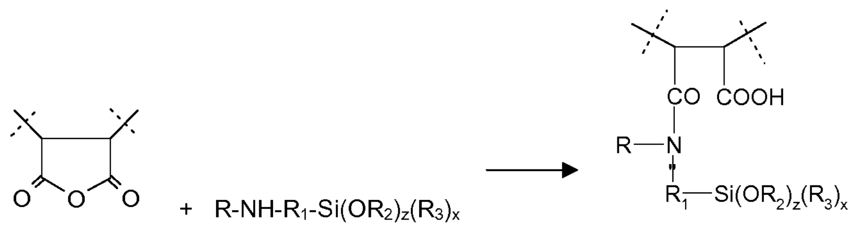

Unité amide et acide carboxylique

Un tel polymère à groupe amino alcoxysilane est nouveau et fait donc également l'objet de la présente invention.
L'invention a aussi pour objet une composition anhydre comprenant un tel polymère à groupe amino alcoxysilane et un milieu physiologiquement acceptable.
Le polymère à groupe amino alcoxysilane est ainsi susceptible d'être obtenu par réaction de l'amino alcoxysilane (I) avec le polymère acrylique d'anhydride maléique décrit précédemment. Une partie ou la totalité des groupes anhydride réagissent avec le groupe NH du composé (I) et forme une unité ayant un groupement amide et un groupe acide carboxylique telle que décrite précédemment.

Selon un mode de réalisation du procédé selon l'invention, on réalise un mélange, notamment extemporané, du polymère acrylique d'anhydride maléique et d'un amino alcoxysilane (I) et le mélange est appliqué sur les matières kératiniques, en particulier sur la peau. On peut aussi effectuer une application séquentielle d'un part du polymère acrylique d'anhydride maléique et d'autre part d'un amino alcoxysilane (I) tels que définis précédemment.

La composition utilisée selon l'invention est généralement adaptée à une application topique sur les matières kértainiques, en particulier sur la peau et comprend donc généralement un milieu physiologiquement acceptable, c'est-à-dire un milieu compatible avec la peau et/ou ses phanères. Il s'agit de préférence d'un milieu cosmétiquement acceptable, c'est-à-dire qui présente une couleur, une odeur et un toucher agréables et qui ne génère pas d'inconforts inacceptables (picotements, tiraillements, rougeurs), susceptibles de détourner la consommatrice d'utiliser cette composition.

Selon un mode préféré de réalisation de l'invention, la composition comprenant le polymère acrylique d'anhydride maléique peut contenir une huile hydrocarbonée.
L'huile hydrocarbonée est une huile liquide à température ambiante (25 °C).
Par huile hydrocarbonée, on entend une huile formée essentiellement, voire constituée, d'atomes de carbone et d'hydrogène, et éventuellement d'atomes d'oxygène, d'azote, et ne contenant pas d'atome de silicium ou de fluor. Elle peut contenir des groupes alcool, ester, éther, acide carboxylique, amine et/ou amide.

L'huile hydrocarbonée peut être volatile ou non volatile.

L'huile hydrocarbonée peut être choisie parmi :

les huiles hydrocarbonées ayant de 8 à 14 atomes de carbone, et notamment :

- les alcanes ramifiés en $C_8$-$C_{14}$ comme les isoalcanes en $C_8$-$C_{14}$ d'origine pétrolière (appelées aussi isoparaffines) comme l'isododécane (encore appelé 2,2,4,4,6-pentaméthylheptane), l'isodécane, et par exemple les huiles vendues sous les noms commerciaux d'Isopars' ou de Permetyls,
- les alcanes linéaires, par exemple tels que le n-dodécane (C12) et le n-tétradécane (C14) vendus par Sasol respectivement sous les références PARAFOL 12-97 et PARAFOL 14-97, ainsi que leurs mélanges, le mélange undécane-tridécane, les mélanges de n-undécane (C11) et de n-tridécane (C13) obtenus aux exemples 1 et 2 de la demande WO2008/155059 de la Société Cognis, et leurs mélanges.

les esters à chaîne courte (ayant de 3 à 8 atomes de carbone au total) tels que l'acétate d'éthyle, l'acétate de

méthyle, l'acétate de propyle, l'acétate de n-butyle

- les huiles hydrocarbonées d'origine végétale telles que les triglycérides constitués d'esters d'acides gras et de glycérol dont les acides gras peuvent avoir des longueurs de chaînes variées de $C_4$ à $C_{24}$, ces dernières pouvant être linéaires ou ramifiées, saturées ou insaturées ; ces huiles sont notamment des triglycérides d'acide heptanoïque ou d'acide octanoïque, ou bien encore les huiles de germe de blé, de tournesol, de pépins de raisin, de sésame, de maïs, d'abricot, de ricin, de karité, d'avocat, d'olive, de soja, d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; le beurre de karité ; ou encore les triglycérides des acides caprylique/caprique comme ceux vendus par la société Stéarineries Dubois ou ceux vendus sous les dénominations Miglyol 810®, 812® et 818® par la société Dynamit Nobel,

- les éthers de synthèse ayant de 10 à 40 atomes de carbone ;

- les hydrocarbures linéaires ou ramifiés, d'origine minérale ou synthétique tels que la vaseline, les polydécènes, le polyisobutène hydrogéné tel que le Parleam ®, le squalane , les huiles de paraffine, et leurs mélanges,

- les esters de synthèse comme les huiles de formule $R_1COOR_2$ dans laquelle $R_1$ représente le reste d'un acide gras linéaire ou ramifié comportant de 1 à 40 atomes de carbone et $R_2$ représente une chaîne hydrocarbonée notamment ramifiée contenant de 1 à 40 atomes de carbone à condition que $R_1 + R_2$ soit $\geq 10$, comme par exemple l'huile de Purcellin (octanoate de cétostéaryle), le myristate d'isopropyle, le palmitate d'isopropyle, les benzoates d'alcools en $C_{12}$ à $C_{15}$, le laurate d'hexyle, l'adipate de diisopropyle, l'isononanoate d'isononyle, le palmitate de 2-éthyl-hexyle, l'isostéarate d'isostéaryle, le laurate de 2-hexyl-décyle, le palmitate de 2-octyl-décyle, le myristate de 2-octyl-dodécyle, des heptanoates, octanoates, décanoates ou ricinoléates d'alcools ou de polyalcools comme le dioctanoate de propylène glycol ; les esters hydroxylés comme le lactate d'isostéaryle, le malate de di-isostéaryle, le lactate de 2-octyl-dodécyle ; les esters de polyols et les esters du pentaérythritol,

- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, et le 2-undécylpentadécanol.

Avantageusement, l'huile hydrocarbonée est apolaire (donc formée uniquement d'atomes de carbone et d'hydrogène).
L'huile hydrocarbonée est de préférence choisie parmi les huiles hydrocarbonées ayant de 8 à 14 atomes de carbone, en particulier les huiles apolaire, décrites précédemment.
Préférentiellement, l'huile hydrocarbonée est l'isododécane.
La composition comprenant le polymère peut contenir, en plus de l'huile hydrocarbonée, une huile siliconée. On entend par « huile siliconée », une huile comprenant au moins un atome de silicium, et notamment au moins un groupe Si-O.
L'huile siliconée peut être volatile ou non volatile.
On entend par « huile volatile », une huile (ou milieu non aqueux) susceptible de s'évaporer au contact de la peau en moins d'une heure, à température ambiante et à pression atmosphérique. L'huile volatile est une huile cosmétique volatile, liquide à température ambiante, ayant notamment une pression de vapeur non nulle, à température ambiante et à pression atmosphérique, en particulier, ayant une pression de vapeur allant de 0,13 Pa à 40 000 Pa ($10^{-3}$ à 300 mm Hg), et de préférence, allant de 1,3 Pa à 13 000 Pa (0,01 à 100 mm Hg), et préférentiellement allant de 1,3 Pa à 1300 Pa (0,01 à 10 mm Hg).
On entend par « huile non volatile », une huile ayant une pression de vapeur inférieure à 0,13 Pa.
Comme huiles siliconées volatiles, on peut citer les huiles de silicones linéaires ou cycliques volatiles, notamment, celles ayant une viscosité $\leq$ 8 centistokes (cSt) ($8 \times 10^{-6}$ $m^2/s$), et ayant, notamment, de 2 à 10 atomes de silicium, et en particulier, de 2 à 7 atomes de silicium, ces silicones comportant, éventuellement, des groupes alkyle ou alkoxy ayant de 1 à 10 atomes de carbone. Comme huile de silicone volatile utilisable dans l'invention, on peut citer, notamment, les diméthicones de viscosité 5 et 6 cSt, l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane, et leurs mélanges.
Comme huiles siliconées non volatiles, on peut citer les polydiméthylsiloxanes (PDMS) non volatiles, linéaires ou cycliques; les polydiméthylsiloxanes comportant des groupements alkyle, alcoxy ou phényle, pendant ou en bout de chaîne siliconée, groupements ayant de 2 à 24 atomes de carbone; les silicones phénylées comme les phényl triméthicones, les phényl diméthicones, les phényl triméthylsiloxy diphényl siloxanes, les diphényl diméthicones, les diphényl

méthyldiphényl trisiloxanes, les 2-phényl éthyl triméthyl-siloxysilicates.

Avantageusement, la composition peut comprendre une huile hydrocarbonée en un teneur allant de 60 à 100 % en poids du poids total des huiles présentes dans la composition et de 0 à 40 % en poids d'huile siliconée. Selon un mode préféré de l'invention, la composition contient comme huile uniquement une huile hydrocarbonée.

La composition selon l'invention peut comprendre un additif cosmétique choisi parmi les parfums, les conservateurs, les charges, les filtres UV, les huiles, les cires, les tensioactifs, les hydratants, les vitamines, les céramides, les antioxydants, les agents anti radicaux libres, les polymères, les épaississants, les matières colorantes.

La composition selon l'invention peut également comprendre une matière colorante comme les matières colorantes pulvérulentes, les colorants liposolubles, les colorants hydrosolubles. Cette matière colorante peut être présente en une teneur allant de 0,01 % à 30 % en poids, par rapport au poids total de la composition.

Les matières colorantes pulvérulentes peuvent être choisies parmi les pigments et les nacres.

Les pigments peuvent être blancs ou colorés, minéraux et/ou organiques, enrobés ou non. On peut citer, parmi les pigments minéraux, le dioxyde de titane, éventuellement traité en surface, les oxydes de zirconium, de zinc ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les nacres peuvent être choisies parmi les pigments nacrés blancs tels que le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les colorants liposolubles sont par exemple le rouge Soudan, le D&C Red 17, le D&C Green 6, le β-carotène, l'huile de soja, le brun Soudan, le D&C Yellow 11, le D&C Violet 2, le D&C orange 5, le jaune quinoléine, le rocou. Les colorants hydrosolubles sont par exemple le jus de betterave, le bleu de méthylène.

Avantageusement, la composition selon l'invention est une composition de soin de la peau.

Avantageusement, la composition selon l'invention est une composition de soin de la peau.

La composition selon l'invention peut être une composition de maquillage tel qu'un fond de teint, un rouge à lèvres, un liner.

Selon un mode de réalisation, la composition selon l'invention est une composition de maquillage et comprend une huile volatile et une huile non volatile telles que décrites précédemment. En particulier, la composition de maquillage peut comprendre une huile volatile hydrocarbonée et une huile non volatile hydrocarbonée.

Selon un mode de réalisation, la composition selon l'invention est une composition de fixation des cheveux.

Selon un mode de réalisation, la composition selon l'invention est une composition anhydre. On entend par composition anhydre une composition contenant moins de 2 % en poids d'eau, voire moins de 0,5 % d'eau, et notamment exempte d'eau. Le cas échéant, d'aussi faibles quantités d'eau peuvent notamment être amenées par des ingrédients de la composition qui peuvent en contenir des quantités résiduelles.

Bien entendu, l'homme du métier veillera à choisir ce ou ces éventuels composés additionnels et/ou leur quantité de manière telle que les propriétés anti-rides de la composition selon l'invention ne soient pas, ou substantiellement pas, altérées par l'adjonction envisagée.

Selon un premier mode de réalisation du procédé selon l'invention, on applique sur les matières kératiniques, notamment sur la peau, un mélange, notamment extemporané, d'une composition cosmétique anhydre comprenant le polymère acrylique d'anhydride maléique et d'un amino alcoxysilane (I) tel que décrits précédemment. Le mélange extemporané est avantageusement effectué moins de 5 minutes avant son application sur les matières kératiniques, notamment sur la peau, et de préférence moins de 3 minutes.

Selon un deuxième mode de réalisation du procédé selon l'invention, on applique sur les matières kératiniques, notamment sur la peau, d'abord une composition anhydre, notamment cosmétique, comprenant le polymère acrylique, puis on applique un amino alcoxysilane (I) ou une composition anhydre cosmétique le contenant. L'application du composé aminé peut être effectuée après un temps compris entre 5 minutes et une heure après avoir appliqué le polymère acrylique sur les matières kératiniques, notamment sur la peau.

Selon un troisième mode de réalisation du procédé selon l'invention, on applique d'abord sur les matières kératiniques, notamment sur la peau, l'amino alcoxysilane (I), ou une composition anhydre cosmétique le contenant, puis on applique la composition anhydre, notamment cosmétique, comprenant le polymère acrylique. L'application du polymère acrylique peut être effectuée après un temps compris entre 5 minutes et une heure après avoir appliqué l'amino alcoxysilane sur les matières kératiniques, notamment sur la peau.

L'application de la composition cosmétique utilisé selon l'invention se fait selon les techniques habituelles, par exemple par application (notamment de crèmes, de gels, de sérums, de lotions) sur la peau destinée à être traitée, en particulier la peau du visage et/ou du cou, notamment la peau du contour de l'oeil. Dans le cadre de ce procédé, la composition peut être, par exemple, une composition de soin.

L'invention va maintenant être décrite en référence aux exemples suivants donnés à titre illustratif et non limitatif.

**Exemple 1** : Copolymère acrylate d'isobornyle / acrylate de 2-éthyl hexyle / anhydride maléique (70/20/10 en poids)

Dans un réacteur double enveloppe de 1 litre muni d'une ancre d'agitation on a introduit 70 g d'acrylate d'isobornyle, 20 g d'acrylate de 2-éthyl hexyle et 10 g d'anhydride maléique. Un mélange de 70 g d'isododécane et de 30 g d'acétate d'éthyle a été ensuite ajouté. Le milieu a été porté à une température de 40°C sous agitation (150 tours/min) et un bullage d'argon a été effectué pendant 10 minutes, puis on a ajouté 0.5g d'amorceur tert-Butyl peroxy-2-ethylhexanoateTrigonox 21S (Trigonox® 21S de chez Akzo Nobel)

Le chauffage de la double enveloppe a été réglé sur 90°C pendant 7 heures à 150 tours/min.
Le milieu a ensuite été dilué avec 300 g d'isododécane, puis concentré par distillation pour éliminer l'acétate d'éthyle et l'anhydride maléique qui n'a pas réagi. On a obtenu au final une solution à 30 % en poids du copolymère dans l'isododécane (rendement supérieur à 90 %).
Le polymère obtenu a un poids moléculaire (Mw) proche de 200 000 g/mol.

**Exemple 2** : Copolymère acrylate d'isobornyle / acrylate de 2-éthyl hexyle / anhydride maléique (65/25/10 en poids)

Le polymère a été préparé selon le mode opératoire de l'exemple 1 en utilisant 65 g d'acrylate d'isobornyle, 25 g d'acrylate de 2-éthyl hexyle et 10 g d'anhydride maléique.
On a obtenu au final une solution à 30 % en poids du copolymère dans l'isododécane (rendement supérieur à 90 %).
Le polymère obtenu a un poids moléculaire (Mw) proche de 200 000g/mol.

**Exemple 3** : Copolymère acrylate d'isobornyle / acrylate de 2-éthyl hexyle / anhydride maléique (75/20/5 en poids)

Le polymère a été préparé selon le mode opératoire de l'exemple 1 en utilisant 75 g d'acrylate d'isobornyle, 20 g d'acrylate de 2-éthyl hexyle et 10 g d'anhydride maléique.
On a obtenu au final une solution à 30 % en poids du copolymère dans l'isododécane (rendement supérieur à 90 %).
Le polymère obtenu a un poids moléculaire (Mw) proche de 200 000g/mol.

**Exemple 4** : Copolymère acrylate d'isobornyle / acrylate de 2-éthyl hexyle / anhydride maléique (60/20/20 en poids)

Le polymère a été préparé selon le mode opératoire de l'exemple 1 en utilisant 60 g d'acrylate d'isobornyle, 20 g d'acrylate de 2-éthyl hexyle et 20 g d'anhydride maléique.
On a obtenu au final une solution à 36 % en poids du copolymère dans l'isododécane (rendement supérieur à 90 %).
Le polymère obtenu a un poids moléculaire (Mw) proche de 200 000 g/mol.

**Exemple 5** :

**Mise en évidence de l'effet tenseur des polymères utilisés selon l'invention**

Cet essai consiste à comparer *in vitro* le pouvoir tenseur du polymère à évaluer par rapport à un polymère tenseur de référence : Hybridur® 875 polymer dispersion de chez Air Products (dispersion aqueuse à 40 % en poids de particules d'un réseau interpénétré de polymères polyuréthane et acrylique). Le polymère à évaluer a été déposé sur une bandelette de caoutchouc nitrile découpée dans un gant vendu sous la référence « Safeskin Nitrile Criticial » n° 038846 par la société Dominique Dutscher SA, d'une surface de 3,5 $cm^2$ préalablement tendues sur un support. Une solution aqueuse contenant le polymère à évaluer est donc déposée sur la bandelette d'élastomère, en déposant 1,8 mg (en matières sèches) de polymère.

On a déposé ainsi sur une bandelette de caoutchouc nitrile 26 $\mu$l d'une solution aqueuse contenant 7 % MA de polymère Hybridur® 875 pour obtenir ainsi une bandelette de référence tenseur et sur une autre bandelette on a déposé 26 $\mu$l d'une solution contenant 7 % MA de polymère acrylique à évaluer dans un mélange isododécane/éthanol (70/30 poids/poids).
Après séchage pendant 24 heures à la température ambiante (25 °C), on observé le recourbement (rétractation) de la bandelette traitée avec le polymère acrylique en comparaison avec celui obtenu avec le témoin (Hybridur® 875).

On a également évalué l'effet tenseur du polymère acrylique en présence de 3-aminopropyltriéthoxysilane (APTES). On a mélangé la solution de polymère acrylique (à 1,75 % MA) et l'APTES dans les proportions indiquées dans le tableau ci-après puis on a déposé une quantité du mélange préparé sur les bandelettes de caoutchouc nitrile.

Polymère de l'exemple 1 :

| Exemple | Proportion d'aptes (équivalent molaire) par rapport aux groupes anhydride du polymère | Quantité d'aptes ajouté (en g) | Quantité de solution du polymère de l'ex 1 (g) | Volume du mélange à prélever pour une bandelette |
|---|---|---|---|---|
| 1a | 0,5 | 0,0355 | 1 | 26 µl |
| 1b | 1 | 0,0711 | 1 | 26 µl |

On a mesuré l'effet tenseur obtenu selon le protocole décrit précédemment. Puis on a évalué la résistance à l'eau de l'effet tenseur en plongeant les bandelettes de caoutchouc traitées avec le polymère à évaluer dans l'eau à la température ambiante 25 °C) pendant 10 minutes, puis en évaluant l'effet tenseur après 1 heure de séchage.

On a obtenu les résultats suivants :

| Polymère testé | Effet tenseur | Effet tenseur après immersion dans l'eau |
|---|---|---|
| Référence Hybridure 875 | correct | Correct |
| Exemple 1 | supérieur à la référence | supérieur à la référence |
| Exemple 1a | supérieur à la référence | supérieur à la référence |
| Exemple 1b | supérieur à la référence | supérieur à la référence |

Les résultats obtenus montrent que le polymère de l'exemple 1 utilisé seul forme un film qui présente un bon effet tenseur, y compris après immersion dans l'eau. La performance tenseur est améliorée en présence d'aptes selon les exemples 1 a et 1 b.

Polymère de l'exemple 2 :

| Exemple | Proportion d'aptes (équivalent molaire) par rapport aux groupes anhydride du polymère | Quantité d'aptes ajouté (en g) | Quantité de solution du polymère de l'ex 2 (g) | Volume du mélange à prélever pour une bandelette |
|---|---|---|---|---|
| 2a | 0,5 | 0,0261 | 1 | 26 µl |
| 2b | 1 | 0,0521 | 1 | 26 µl |
| 2c | 2 | 0,1043 | 1 | 26 µl |

On a obtenu les résultats suivants :

| Polymère testé | Effet tenseur | Effet tenseur après immersion dans l'eau |
|---|---|---|
| Référence Hybridure 875 | correct | Correct |
| Exemple 2 | comparable à la référence | supérieur à la référence |
| Exemple 2a | supérieur à la référence | supérieur à la référence |
| Exemple 2b | supérieur à la référence | supérieur à la référence |
| Exemple 2c | comparable à la référence | comparable à la référence |

Les résultats obtenus montrent que le polymère de l'exemple 2 utilisé seul forme un film qui présente un bon effet tenseur, y compris après immersion dans l'eau. La performance tenseur est améliorée en présence d'aptes selon les exemples 2 a, 2b et 2 c.

Polymère de l'exemple 3 :

| Exemple | Proportion d'aptes (équivalent molaire) par rapport aux groupes anhydride du polymère | Quantité d'aptes ajouté (en g) | Quantité de solution du polymère de l'ex 3 (g) | Volume du mélange à prélever pour une bandelette |
|---|---|---|---|---|
| 3a | 0,5 | 0,0263 | 1 | 26 μl |
| 3b | 1 | 0,0526 | 1 | 26 μl |
| 3c | 2 | 0,1052 | 1 | 26 μl |

On a obtenu les résultats suivants :

| **Polymère testé** | **Effet tenseur** | **Effet tenseur après immersion dans l'eau** |
|---|---|---|
| Référence Hybridure 875 | correct | Correct |
| Exemple 3 | comparable à la référence | supérieur à la référence |
| Exemple 3a | comparable à la référence | supérieur à la référence |
| Exemple 3b | comparable à la référence | supérieur à la référence |
| Exemple 3c | comparable à la référence | supérieur à la référence |

Les résultats obtenus montrent que le polymère de l'exemple 3 utilisé seul forme un film qui présente un bon effet tenseur, y compris après immersion dans l'eau. La performance tenseur, après immersion dans l'eau, est améliorée en présence d'aptes selon les exemples 3 a, 3b et 3 c.

**Exemple 6** :

On prépare un gel anti-rides ayant la composition suivante :

| | |
|---|---|
| - polymère de l'exemple 1 en solution à 30 % en poids dans l'isododécane | 7 g MA |
| - disteardimonium hectorite/ppropylene carbonate dans isodoecane (bentone gel ® ISDV de chez Elementis) | 3 g |
| - Conservateurs | qs |
| - Isododecane/éthanol (80/20 p/p) qsp | 100 g |

Juste avant l'application sur la peau, on ajoute dans le gel 1,58 g de 3-aminopropyltriethoxysilane (APTES).

Une composition similaire est également préparée en utilisant le polymère de l'exemple 2 ou 3 ou 4.
La composition obtenue appliquée sur le visage permet de lisser efficacement les rides.

**Exemple 7 à 14** : **Evaluation cosmétique de compositions de maquillage**

On a préparé les 8 compositions de maquillage de base coat et de top coat décrites ci-dessous (exemples 8, 10, 12, 14 selon l'invention : top coat avec APTES ; exemples 7, 9, 11, 13 hors invention : top coat sans APTES)

On a appliqué chaque composition de base coat sur un support équivalent de peau en élastomère en réalisant un dépôt d'une épaisseur de 100 μm humide et on a laissé sécher à température ambiante (25 °C) pendant 24 heures.

Puis on a appliqué sur chaque dépôt de base coat sec la composition de top coat en réalisant un dépôt d'une épaisseur de 100 μm humide et on a laissé sécher à température ambiante (25 °C) pendant 24 heures.

On a ensuite observé l'état du film obtenu.

On a évalué la résistance du film obtenu en appliquant séparément 0,5 ml d'huile d'olive et 0,5 ml de sébum ; après 5 minutes de contact on a frotté la surface du film avec un coton puis on a observé l'état du film

On a également évalué l'aspect collant du film et son aptitude à transférer ou non en touchant le film avec le doigt

L'évaluation a été faite de la façon suivante :

+++ : propriété cosmétique évaluée très performante
++ : propriété cosmétique évaluée moyennement performante
+ : propriété cosmétique évaluée peu performante
o : propriété cosmétique évaluée non performante

On a obtenu les résultats suivants :

| | Exemple 7 | Exemple 8 (invention) | Exemple 9 | Exemple 10 (invention) |
|---|---|---|---|---|
| **Base Coat** | | | | |
| Polymère de l'exemple 4 | 20g | 20 g | 20 g | 20 g |
| Pâte pigmentaire à 40 % en poids de pigment dans l'isododécane | 5g avec DCRed 7 | 5g avec DCRed 7 | 5g avec DCRed 7 | 5g avec DCRed 7 |
| Disteardimonium Hectorite (BENTONE GEL ISD V de chez Elementis) | 10g | 10g | 10g | 10g |
| Isododécane | 65g | 65g | 45g | 45g |
| Octyl-2 dodécanol | | | 20g | 20g |
| **Top Coat** | | | | |
| **APTES** | | **10g** | | **10g** |
| Isododécane | | 90g | | 90g |
| Aspect du film | Film homogène | Film homogène | Film homogène | Film homogène |
| Résistance à l'huile d'olive | + | +++ | + | +++ |
| Résistance au sébum | + | +++ | + | +++ |
| Non collant | +++ | +++ | +++ | +++ |
| Non transfert | +++ | +++ | +++ | +++ |

| | Exemple 11 | Exemple 12 (invention) | Exemple 13 | Exemple 14 (invention) |
|---|---|---|---|---|
| **Base Coat** | | | | |
| Polymère de l'exemple 4 | 20g | 20 g | 20g | 20g |
| Pâte pigmentaire 40 % en poids de pigment dans l'isododécane | 5g avec oxyde de fer noir | 5g avec oxyde de fer noir | 5g avec oxyde de fer noir | 5g avec oxyde de fer noir |
| Disteardimonium Hectorite (BENTONE GEL ISD V de chez Elementis) | 10g | 10g | 10g | 10g |
| Isododécane | 65g | 65g | 45g | 45g |
| Octyl-2 dodécanol | | | 20g | 20g |
| **Top Coat** | | | | |
| **APTES** | | **10g** | | **10g** |
| Isododécane | | 90g | | 90g |
| Aspect du film | Film homogène | Film homogène | Film homogène | Film homogène |
| Résistance à l'huile d'olive | + | +++ | + | +++ |
| Résistance au sébum | + | +++ | + | +++ |

(suite)

| **Top Coat** | | | | |
|---|---|---|---|---|
| Non collant | +++ | +++ | +++ | +++ |
| Non transfert | +++ | +++ | +++ | +++ |

Les résultats obtenus montrent que les dépôts résultant de l'application du polymère 4, avec ou sans octyl-2 dodécanol, puis de l'Aptes (exemples 8, 10 ; 12, 14) forment un film homogène non collant et ne transférant pas au doigt, et résistant à l'huile et au sébum tandis que la seule application du polymère 4 (exemples 7, 9 ; 11, 13) forme un dépôt présentant une plus faible résistance à l'huile et au sébum.
Ainsi, la résistance du film au contact de l'huile d'olive et du sébum est nettement améliorée avec l'application de la composition top coat contenant l'APTES.

**Exemple 15 : Evaluation cosmétique de composition capillaire**

Sur une mèche de cheveux lavée et séchée de 2,7 g (mèche n° 1) on a appliqué 0,5 g d'une composition base coat contenant 10 % MA du polymère de l'exemple 1 dans l'isododécane. On a laissé séché la mèche traitée à l'air libre (25 °C) pendant 24 heures.
Puis on a appliqué sur la mèche 0,5 g d'une composition top coat contenant 10 % MA d'APTES dans l'isododécane, puis laissé séché à l'air libre pendant 24 heures.
On a ensuite évalué la rémanence à l'eau de la mèche traitée de la propriété de fixation en immergeant dans l'eau la mèche traitée pendant 5 minutes. On a ensuite essoré la mèche puis on les a séchées au casque. On a observé la rigidité de la mèche par comparaison avec une mèche non traitée.
On a constaté que la mèche traitée avant et après immersion dans l'eau présente une forme rigide avec une bonne fixation des cheveux. La fixation des cheveux présente donc une bonne rémanence à l'eau.

**Exemple 16 : Evaluation cosmétique de composition de mascara**

Sur une éprouvette de faux cils, on a appliqué une composition de base coat contenant 20 % MA de polymère de l'exemple 4, 5 % d'oxyde de fer rouge, 10 % de Disteardimonium Hectorite (BENTONE GEL ISD V de chez Elementis), 65 % d'isododécane. On a laissé séché les cils traités à l'air libre (25 °C) pendant 24 heures. Puis on a appliqué sur la mèche 0,5 g d'une composition top coat contenant 10 % MA d'APTES dans l'isododécane, puis laissé séché à l'air libre pendant 24 heures.
On a ensuite évalué la rémanence au sébum du dépôt formé sur les cils traités en immergeant dans du sébum artificiel les cils traités pendant 5 minutes. On a ensuite séché les cils à l'air libre et on a frotté les cils contre un papier buvard. On a observé aucune trace de dépôt sur le papier : le dépôt formé sur les cils est donc bien résistant au sébum.

## Revendications

**1.** Procédé, notamment cosmétique, de soin ou de maquillage des matières kératiniques comprenant :

soit l'application topique sur les matières kératiniques d'un mélange anhydre (extemporané) d'une composition cosmétique comprenant un polymère acrylique d'anhydride maléique et d'un composé amino alcoxysilane (I), ou d'une composition cosmétique le contenant ;
soit l'application séquentielle sur les matières kératiniques d'une composition cosmétique anhydre comprenant un polymère acrylique d'anhydride maléique et d'un composé amino alcoxysilane (I), ou d'une composition cosmétique anhydre le contenant,
ledit polymère acrylique d'anhydride maléique étant susceptible d'être obtenu par polymérisation de :

(a) 50 à 90 % en poids, du poids total de monomères, de (méth)acrylate d'isobornyle
(b) 1 à 50 % en poids d'anhydride maléique
(c) 0 à 49 % en poids de monomère (méth)acrylate additionnel choisi parmi :

(i) les (métha)crylate d'alkyle en $C_1$-$C_{20}$, linéaire ou ramifié, saturé ou insaturé, éventuellement interrompu par un ou plusieurs hétéroatomes non adjacents choisi parmi O ou S ou par un groupement

NR , R étant un groupe alkyle en $C_1$-$C_4$, éventuellement substitué par un groupement phényle ou furfuryle ;
(ii) les (méth)acrylate de cycloalkyle en $C_4$-$C_8$ saturé éventuellement interrompu par O ou NH ;

ledit amino alcoxy silane étant de formule (I) :

$$R\text{-}NH\text{-}R_1Si(OR_2)_z(R_3)_x \qquad (I)$$

dans laquelle :

$R_1$ est un groupe divalent hydrocarbonée en $C_1$-$C_{20}$, linéaire ou ramifiée, saturée ou insaturée, cyclique ou acyclique, pouvant être interrompu dans sa chaine par un hétéroatome (O, S, NH) ou un groupement carbonyle (CO), $R_1$ étant lié à l'atome de silicium directement via un atome de carbone ;
R= H ou groupe alkyle en $C_1$-$C_4$, de préférence H ;
$R_2$ et $R_3$ identiques ou différents, représentent un groupe alkyle, linéaire ou ramifié, comprenant de 1 à 6 atomes de carbone,
z désigne un nombre entier allant de 1 à 3, et
x désigne un nombre entier allant de 0 à 2,
avec z+x =3.

**2.** Procédé selon la revendication 1, **caractérisé en ce que** ledit polymère acrylique d'anhydride maléique est issu de la polymérisation de :

(a) 50 à 80 % en poids, du poids total de monomères, de (méth)acrylate d'isobornyle
(b) 5 à 30 % en poids d'anhydride maléique.
(c) 15 à 30 % en poids dudit monomère (méth)acrylate additionnel.

**3.** Procédé selon l'une des revendications précédentes, **caractérisé en ce que** ledit polymère acrylique d'anhydride maléique est issu de la polymérisation de :

(a) 60 à 80 % en poids, du poids total de monomères, de (méth)acrylate d'isobornyle
(b) 5 à 25 % en poids d'anhydride maléique.
(c) 15 à 30 % en poids dudit monomère (méth)acrylate additionnel.

**4.** Procédé selon l'une des revendications précédentes, **caractérisé en ce que** le polymère acrylique d'anhydride maléique est issu de la polymérisation de :

(a) 50 à 80 % en poids, du poids total de monomères, de (méth)acrylate d'isobornyle
(b) 5 à 15 % en poids d'anhydride maléique
(c) 15 à 30 % en poids dudit monomère (méth)acrylate additionnel.

**5.** Procédé selon l'une des revendications précédentes, **caractérisé en ce que** le polymère acrylique d'anhydride maléique est issu de la polymérisation de :

(a) 60 à 80 % en poids, du poids total de monomères, de (méth)acrylate d'isobornyle
(b) 5 à 12 % en poids d'anhydride maléique.
(c) 18 à 30 % en poids dudit monomère (méth)acrylate additionnel.

**6.** Procédé selon l'une des revendications précédentes, **caractérisé en ce que** ledit monomère (méth)acrylate additionnel est choisi parmi les (méth)acrylate d'alkyle en $C_6$-$C_{16}$, et de préférence parmi les acrylate d'alkyle en $C_6$-$C_{16}$.

**7.** Procédé selon l'une des revendications précédentes, **caractérisé en ce que** ledit polymère acrylique d'anhydride maléique est issu de la polymérisation de :

(a) 50 à 80 % en poids, du poids total de monomères, de (méth)acrylate d'isobornyle
(b) 5 à 30 % en poids d'anhydride maléique.
(c) 15 à 30 % en poids de monomère acrylate d'alkyle en $C_6$-$C_{16}$.

**8.** Procédé selon l'une des revendications précédentes, **caractérisé en ce que** le polymère acrylique d'anhydride maléique est issu de la polymérisation de :

(a) 50 à 80 % en poids, du poids total de monomères, de (méth)acrylate d'isobornyle
(b) 5 à 30 % en poids d'anhydride maléique.
(c) 15 à 30 % en poids de monomère acrylate d'alkyle en $C_6$-$C_{16}$,

et de préférence qu'il est issu de la polymérisation de :

(a) 50 à 80 % en poids, du poids total de monomères, de (méth)acrylate d'isobornyle
(b) 5 à 15 % en poids d'anhydride maléique.
(c) 15 à 30 % en poids de monomère acrylate d'alkyle en $C_6$-$C_{16}$.

**9.** Procédé selon l'une des revendications précédentes, **caractérisé en ce que** la polymère acrylique comprend de l'acrylate d'isobornyle, l'acrylate de 2-éthylhexyle et l'anhydride maléique.

**10.** Procédé selon l'une quelconque des revendications précédentes, **caractérisé en ce que** le polymère acrylique a un poids moléculaire moyen en poids allant de 5000 à 1 000 000 g/mole de préférence allant de 10000 à 500 000 g/mole, et préférentiellement allant de 15000 à 350 000 g/mole.

**11.** Procédé selon l'une quelconque des revendications précédentes, **caractérisé en ce que** pour l'amino alcoxysilane (I) mis en oeuvre dans le procédé selon l'invention :

$$R = H ;$$

$R_1$ est une chaîne hydrocarbonée en $C_1$-$C_6$, linéaire saturée, de préférence en $C_2$-$C_4$ ;
$R_2$ représente un groupe alkyle comprenant de 1 à 4 atomes de carbone, de préférence linéaire, de préférence représente le groupe éthyle ;
$R_3$ représente un groupe alkyle comprenant de 1 à 4 atomes de carbone, de préférence linéaire, de préférence représente un groupe méthyle ou éthyle ;

de préférence :

$R_1$ est une chaîne hydrocarbonée en $C_1$-$C_6$, linéaire saturée substituée,

$$R = H ,$$

$R_2$ représente un groupe alkyle comprenant de 1 à 4 atomes de carbone,
$R_3$ représente un groupe alkyle comprenant de 1 à 4 atomes de carbone.

**12.** Procédé selon l'une quelconque des revendications précédentes, **caractérisé en ce que** l'amino alcoxysilane (I) est choisi parmi le 3-aminopropyltriéthoxysilane, le 3-aminoéthyltriéthoxysilane, le 3-aminopropylméthyldiéthoxysilane, le N-(2-aminoéthyl)-3-aminopropyltriéthoxysilane, le 3-(m-aminophénoxy)propyltriméthoxysilane, le p-aminophényltriméthoxysilane, le N-(2-aminoéthylaminométhyl)phénéthyltriméthoxysilane ;
de préférence choisi parmi le 3-aminopropyltriéthoxysilane (APTES), le 3-aminoéthyltriéthoxysilane (AETES), le 3-aminopropylméthyldiéthoxysilane, le N-(2-aminoéthyl)-3-aminopropyltriéthoxysilane ; préférentiellement est le 3-aminopropyl triéthoxysilane.

**13.** Procédé selon l'une quelconque des revendications précédentes, **caractérisé en ce que** l'amino alcoxysilane (I) est mis en oeuvre selon un ratio molaire amino alcoxysilane / groupe anhydride maléique du polymère acrylique allant de 0,01 à 10, de préférence allant de 0,1 à 5, préférentiellement allant de 0,1 à 2, et plus préférentiellement allant de 0,1 à 1.

**14.** Procédé selon l'une quelconque des revendications précédentes, **caractérisé en ce que** le polymère acrylique est présent en une teneur allant de 0,1 à 10 % en poids, par rapport au poids total de la composition, de préférence de 0,5 % à 10 % en poids, et préférentiellement allant de 1 % à 8 % en poids, et plus préférentiellement allant de 1 %

à 6 % en poids.

**15.** Procédé selon l'une quelconque des revendications précédentes, **caractérisé en ce que** la composition comprend une huile hydrocarbonée, de préférence une huile hydrocarbonée apolaire ayant de 8 à 14 atomes de carbone, préférentiellement l'isododécane.

**16.** Procédé selon l'une quelconque des revendications précédentes, **caractérisé par le fait que** l'on applique sur les matières kératiniques un mélange effectué moins de 5 minutes avant l'application sur les matières kératiniques de la composition comprenant le polymère acrylique d'anhydride maléique et de l'amino alcoxysilane ou de la composition le contenant.

**17.** Procédé selon l'une quelconque des revendications 1 à 15, **caractérisé par le fait que** l'on applique sur les matières kératiniques d'abord la composition comprenant le polymère acrylique d'anhydride maléique puis on applique l'amino alcoxysilane (I) ou une composition anhydre le contenant et comprenant un milieu physiologiquement acceptable.

**18.** Procédé selon l'une quelconque des revendications 1 à 15, **caractérisé par le fait que** l'on applique sur les matières kératiniques d'abord l'amino alcoxysilane (I), ou une composition anhydre le contenant et comprenant un milieu physiologiquement acceptable, puis on applique la composition comprenant le polymère acrylique d'anhydride maléique.

**19.** Procédé selon l'une quelconque des revendications précédentes, **caractérisé en ce qu'**il est appliqué sur la peau, plus particulièrement la peau du visage, en particulier la peau ridée.

**20.** Procédé selon l'une quelconque des revendications précédentes, **caractérisé en ce qu'**il est destiné à atténuer les rides.

**21.** Utilisation cosmétique en tant qu'agent tenseur de la peau, en particulier d'une peau ridée, du polymère acrylique d'anhydride maléique tel que défini dans l'une quelconque des revendications 1 à 10 et en mélange avec un amino alcoxysilane (I), ou d'une composition anhydre le contenant et comprenant un milieu physiologiquement acceptable, tel que défini dans l'une des revendications 1 et 11, 12.

**22.** Composition obtenue par mélange d'une composition d'une composition anhydre comprenant un polymère acrylique d'anhydride maléique tel que défini dans l'une quelconque des revendications 1 à 10 et d'un amino alcoxyosilane (I) ou d'une composition anhydre le contenant et comprenant un milieu physiologiquement acceptable, tel que défini dans l'une des revendications 1 et 11, 12.

**23.** Composition selon la revendication précédente, **caractérisée en ce qu'**elle est une composition de maquillage comprenant une huile volatile et une huile non volatile, de préférence une huile volatile hydrocarbonée et une huile non volatile hydrocarbonée.

**24.** Kit comprenant une première composition anhydre comprenant un polymère acrylique d'anhydride maléique tel que défini dans l'une quelconque des revendications 1 à 10 et une deuxième composition anhydre comprenant un amino alcoxysilane (I) tel que défini dans l'une des revendications 1 et 11, 12 et comprenant un milieu physiologiquement acceptable, les première et deuxième compositions étant conditionnées chacune dans un ensemble de conditionnement distinct.

**25.** Polymère susceptible d'être obtenu par réaction d'un amino alcoxysilane (I) tel que défini dans l'une des revendications 1 et 11, 12 avec un polymère acrylique d'anhydride maléique tel que défini dans l'une quelconque des revendications 1 à 10.

## Patentansprüche

**1.** Verfahren, insbesondere kosmetisches Verfahren, zur Pflege oder zum Make-up von Keratinmaterialien, umfassend:

entweder das topische Aufbringen einer (extemporalen) wasserfreien Mischung einer kosmetischen Zusammensetzung, die ein Maleinsäureanhydrid-Acrylpolymer umfasst, und einer Aminoalkoxysilanverbindung (I) oder einer kosmetischen Zusammensetzung, die diese enthält, auf die Keratinmaterialien;

oder das aufeinanderfolgende Aufbringen einer wasserfreien kosmetischen Zusammensetzung, die ein Maleinsäureanhydrid-Acrylpolymer umfasst, und einer Aminoalkoxysilanverbindung (I) oder einer wasserfreien kosmetischen Zusammensetzung, die diese enthält, auf die Keratinmaterialien,
wobei das Maleinsäureanhydrid-Acrylpolymer durch Polymerisation von:

(a) 50 bis 90 Gew.-%, bezogen auf das Gesamtgewicht der Monomere, Isobornyl(meth)acrylat,
(b) 1 bis 50 Gew.-% Maleinsäureanhydrid,
(c) 0 bis 49 Gew.-% zusätzliches (Meth)acrylat-Monomer, ausgewählt aus:

(i) linearen oder verzweigten, gesättigten oder ungesättigten $C_1$-$C_{20}$-Alkyl (meth) acrylaten, die gegebenenfalls durch ein oder mehrere nicht benachbarte Heteroatome, die aus O oder S ausgewählt sind, oder durch eine Gruppe NR unterbrochen sind, wobei R für eine $C_1$-$C_4$-Alkylgruppe, die gegebenenfalls durch eine Phenyl- oder Furfurylgruppe substituiert ist, steht;
(ii) gesättigten $C_4$-$C_8$-Cycloalkyl (meth) acrylaten, die gegebenenfalls durch O oder NH unterbrochen sind;

erhältlich ist;
wobei das Aminoalkoxysilan die Formel (I) aufweist:

$$R\text{-}NH\text{-}R_1Si(OR_2)_z(R_3)_x \qquad (I)$$

in der:

$R_1$ für eine lineare oder verzweigte, gesättigte oder ungesättigte, cyclische oder acyclische zweiwertige $C_1$-$C_{20}$-Kohlenwasserstoffgruppe, die gegebenenfalls in der Kette durch ein Heteroatom (O, S, NH) oder eine Carbonylgruppe (CO) unterbrochen sein kann, steht, wobei $R_1$ über ein Kohlenstoffatom direkt an das Siliciumatom gebunden ist;
R = H oder eine $C_1$-$C_4$-Alkylgruppe, bevorzugt H;
$R_2$ und $R_3$ gleich oder verschieden sind und für eine lineare oder verzweigte Alkylgruppe mit 1 bis 6 Kohlenstoffatomen stehen,
z für eine ganze Zahl im Bereich von 1 bis 3 steht und
x für eine ganze Zahl im Bereich von 0 bis 2 steht,

wobei z + x = 3.

2. Verfahren nach Anspruch 1, **dadurch gekennzeichnet, dass** sich das Maleinsäureanhydrid-Acrylpolymer aus der Polymerisation von:

(a) 50 bis 80 Gew.-%, bezogen auf das Gesamtgewicht der Monomere, Isobornyl(meth)acrylat,
(b) 5 bis 30 Gew.-% Maleinsäureanhydrid,
(c) 15 bis 30 Gew.-% des zusätzlichen (Meth)-acrylat-Monomers

ableitet.

3. Verfahren nach einem der vorhergehenden Ansprüche, **dadurch gekennzeichnet, dass** sich das Maleinsäureanhydrid-Acrylpolymer aus der Polymerisation von:

(a) 60 bis 80 Gew.-%, bezogen auf das Gesamtgewicht der Monomere, Isobornyl(meth)acrylat,
(b) 5 bis 25 Gew.-% Maleinsäureanhydrid,
(c) 15 bis 30 Gew.-% des zusätzlichen (Meth)-acrylat-Monomers

ableitet.

4. Verfahren nach einem der vorhergehenden Ansprüche, **dadurch gekennzeichnet, dass** sich das Maleinsäureanhydrid-Acrylpolymer aus der Polymerisation von:

(a) 50 bis 80 Gew.-%, bezogen auf das Gesamtgewicht der Monomere, Isobornyl(meth)acrylat,
(b) 5 bis 15 Gew.-% Maleinsäureanhydrid,

(c) 15 bis 30 Gew.-% des zusätzlichen (Meth)-acrylat-Monomers

ableitet.

5. Verfahren nach einem der vorhergehenden Ansprüche, **dadurch gekennzeichnet, dass** sich das Maleinsäureanhydrid-Acrylpolymer aus der Polymerisation von:

(a) 60 bis 80 Gew.-%, bezogen auf das Gesamtgewicht der Monomere, Isobornyl(meth)acrylat,
(b) 5 bis 12 Gew.-% Maleinsäureanhydrid,
(c) 18 bis 30 Gew.-% des zusätzlichen (Meth)-acrylat-Monomers

ableitet.

6. Verfahren nach einem der vorhergehenden Ansprüche, **dadurch gekennzeichnet, dass** das zusätzliche (Meth) acrylat-Monomer aus $C_6$-$C_{16}$-Alkyl (meth) - acrylaten und bevorzugt aus $C_6$-$C_{16}$-Alkylacrylaten ausgewählt ist.

7. Verfahren nach einem der vorhergehenden Ansprüche, **dadurch gekennzeichnet, dass** sich das Maleinsäureanhydrid-Acrylpolymer aus der Polymerisation von:

(a) 50 bis 80 Gew.-%, bezogen auf das Gesamtgewicht der Monomere, Isobornyl(meth)acrylat,
(b) 5 bis 30 Gew.-% Maleinsäureanhydrid,
(c) 15 bis 30 Gew.-% $C_6$-$C_{16}$-Alkylacrylat-Monomer ableitet.

8. verfahren nach einem der vorhergehenden Ansprüche, **dadurch gekennzeichnet, dass** sich das Maleinsäureanhydrid-Acrylpolymer aus der Polymerisation von:

(a) 50 bis 80 Gew.-%, bezogen auf das Gesamtgewicht der Monomere, Isobornyl(meth)acrylat,
(b) 5 bis 30 Gew.-% Maleinsäureanhydrid,
(c) 15 bis 30 Gew.-% $C_6$-$C_{16}$-Alkylacrylat-Monomer ableitet

und sich bevorzugt aus der Polymerisation von:

(a) 50 bis 80 Gew.-%, bezogen auf das Gesamtgewicht der Monomere, Isobornyl(meth)acrylat,
(b) 5 bis 15 Gew.-% Maleinsäureanhydrid,
(c) 15 bis 30 Gew.-% $C_6$-$C_{16}$-Alkylacrylat-Monomer ableitet.

9. Verfahren nach einem der vorhergehenden Ansprüche, **dadurch gekennzeichnet, dass** das Acrylpolymer Isobornylacrylat, 2-Ethylhexylacrylat und Maleinsäureanhydrid umfasst.

10. Verfahren nach einem der vorhergehenden Ansprüche, **dadurch gekennzeichnet, dass** das Acrylpolymer ein gewichtsmittleres Molekulargewicht im Bereich von 5000 bis 1,000.000 g/mol, bevorzugt im Bereich von 10.000 bis 500.000 g/mol und vorzugsweise im Bereich von 15.000 bis 350.000 g/mol aufweist.

11. Verfahren nach einem der vorhergehenden Ansprüche, **dadurch gekennzeichnet, dass** für das bei dem erfindungsgemäßen Verfahren verwendete Aminoalkoxysilan (I):

$$R = H;$$

$R_1$ für eine lineare gesättigte $C_1$-$C_6$- und bevorzugt $C_2$-$C_4$-Kohlenwasserstoffkette steht;
$R_2$ für eine Alkylgruppe mit 1 bis 4 Kohlenstoffatomen, die bevorzugt linear ist, steht und bevorzugt für eine Ethylgruppe steht;
$R_3$ für eine Alkylgruppe mit 1 bis 4 Kohlenstoffatomen, die bevorzugt linear ist, steht und bevorzugt für eine Methyl- oder Ethylgruppe steht;

bevorzugt:

$R_1$ für eine substituierte lineare gesättigte $C_1$-$C_6$-Kohlenwasserstoffkette steht,

$R = H$,

$R_2$ für eine Alkylgruppe mit 1 bis 4 Kohlenstoffatomen steht;
$R_3$ für eine Alkylgruppe mit 1 bis 4 Kohlenstoffatomen steht.

**12.** Verfahren nach einem der vorhergehenden Ansprüche, **dadurch gekennzeichnet, dass** das Aminoalkoxysilan (I) aus 3-Aminopropyltriethoxysilan, 3-Aminoethyltriethoxysilan, 3-Aminopropylmethyldiethoxysilan, N-(2-Aminoethyl)-3-aminopropyltriethoxysilan, 3-(m-Aminophenoxy)propyltrimethoxysilan, p-Aminophenyltrimethoxysilan und N-(2-Aminoethylamino-methyl)phenethyltrimethoxysilan und
bevorzugt aus 3-Aminopropyltriethoxysilan (APTES), 3-Aminoethyltriethoxysilan (AETES), 3-Aminopropylmethyldiethoxysilan und N-(2-Aminoethyl)-3-aminopropyltriethoxysilan ausgewählt ist und vorzugsweise 3-Aminopropyltriethoxysilan ist.

**13.** Verfahren nach einem der vorhergehenden Ansprüche, **dadurch gekennzeichnet, dass** das Aminoalkoxysilan (I) in einem Molverhältnis Aminoalkoxysilan/Maleinsäureanhydridgruppe des Acrylpolymers im Bereich von 0,01 bis 10, bevorzugt im Bereich von 0,1 bis 5, vorzugsweise im Bereich von 0,1 bis 2 und bevorzugt im Bereich von 0,1 bis 1 verwendet wird.

**14.** Verfahren nach einem der vorhergehenden Ansprüche, **dadurch gekennzeichnet, dass** das Acrylpolymer in einem Gehalt im Bereich von 0,1 bis 10 Gew.-%, bezogen auf das Gesamtgewicht der Zusammensetzung, bevorzugt von 0,5 bis 10 Gew.-% und vorzugsweise im Bereich von 1 bis 8 Gew.-% und weiter bevorzugt im Bereich von 1 bis 6 Gew.-% vorliegt.

**15.** Verfahren nach einem der vorhergehenden Ansprüche, **dadurch gekennzeichnet, dass** die Zusammensetzung ein Kohlenwasserstofföl, bevorzugt ein unpolares Kohlenwasserstofföl mit 8 bis 14 Kohlenstoffatomen, vorzugsweise Isododecan, umfasst.

**16.** Verfahren nach einem der vorhergehenden Ansprüche, **dadurch gekennzeichnet, dass** man auf die Keratinmaterialien eine weniger als 5 Minuten vor dem Aufbringen auf die Keratinmaterialien hergestellte Mischung der Zusammensetzung, die das Maleinsäureanhydrid-Acrylpolymer umfasst, und des Aminoalkoxysilans oder einer kosmetischen Zusammensetzung, die dieses enthält, aufbringt.

**17.** Verfahren nach einem der Ansprüche 1 bis 15, **dadurch gekennzeichnet, dass** man auf die Keratinmaterialien zunächst die Zusammensetzung, die das Maleinsäureanhydrid-Acrylpolymer umfasst, und dann das Aminoalkoxysilan oder eine kosmetische Zusammensetzung, die dieses enthält und ein physiologisch unbedenkliches Medium umfasst, auf-bringt.

**18.** Verfahren nach einem der Ansprüche 1 bis 15, **dadurch gekennzeichnet, dass** man auf die Keratinmaterialien zunächst das Aminoalkoxysilan oder eine kosmetische Zusammensetzung, die dieses enthält und ein physiologisch unbedenkliches Medium umfasst, und dann die Zusammensetzung, die das Maleinsäureanhydrid-Acrylpolymer umfasst, aufbringt.

**19.** Verfahren nach einem der vorhergehenden Ansprüche, **dadurch gekennzeichnet, dass** es auf die Haut, insbesondere die Gesichtshaut, insbesondere faltige Haut, angewendet wird.

**20.** Verfahren nach einem der vorhergehenden Ansprüche, **dadurch gekennzeichnet, dass** es zur Abschwächung von Falten vorgesehen ist.

**21.** Kosmetische Verwendung von Maleinsäureanhydrid-Acrylpolymer gemäß einem der Ansprüche 1 bis 10 und als Mischung mit einem Aminoalkoxysilan (I) oder einer wasserfreien Zusammensetzung, die dieses enthält und ein physiologisch unbedenkliches Medium umfasst, gemäß einem der Ansprüche 1, 11 und 12 als Straffungsmittel für die Haut, insbesondere für faltige Haut.

**22.** Zusammensetzung, erhalten durch Mischen einer wasserfreien Zusammensetzung, die ein Maleinsäureanhydrid-Acrylpolymer umfasst, gemäß einem der Ansprüche 1 bis 10 und eines Aminoalkoxysilans (I) oder einer wasserfreien kosmetischen Zusammensetzung, die dieses enthält und ein physiologisch unbedenkliches Medium umfasst, gemäß einem der Ansprüche 1, 11 und 12.

**23.** Zusammensetzung nach dem vorhergehenden Anspruch, **dadurch gekennzeichnet, dass** es sich um eine Make-up-Zusammensetzung handelt, die ein flüchtiges Öl und ein nichtflüchtiges Öl, bevorzugt ein flüchtiges Kohlenwasserstofföl und ein nichtflüchtiges Kohlenwasserstofföl, umfasst.

**24.** Kit, umfassend eine erste wasserfreie Zusammensetzung, die ein Maleinsäureanhydrid-Acrylpolymer umfasst, gemäß einem der Ansprüche 1 bis 10 und eine zweite wasserfreie Zusammensetzung, die ein Aminoalkoxysilan (I) gemäß einem der Ansprüche 1, 11 und 12 umfasst und ein physiologisch unbedenkliches Medium umfasst, wobei die erste Zusammensetzung und die zweite Zusammensetzung jeweils in einer separaten Verpackungsanordnung verpackt sind.

**25.** Polymer, das durch Reaktion eines Aminoälkoxysilans (I) gemäß einem der Ansprüche 1, 11 und 12 mit einem Maleinsäure-Acrylpolymer gemäß einem der Ansprüche 1 bis 10 erhältlich ist.

## Claims

**1.** Process, in particular a cosmetic process, for caring for or making up keratin materials, comprising:

either the topical application to the keratin materials of an anhydrous (extemporaneous) mixture of a cosmetic composition comprising a maleic anhydride acrylic polymer and of an amino alkoxysilane compound (I) or of a cosmetic composition containing same;
or the sequential application to the keratin materials of an anhydrous cosmetic composition comprising a maleic anhydride acrylic polymer and of an amino alkoxysilane compound (I) or of an anhydrous cosmetic composition containing same,
said maleic anhydride acrylic polymer being able to be obtained by polymerization of:

(a) 50% to 90% by weight, relative to the total weight of monomers, of isobornyl (meth)acrylate
(b) 1% to 50% by weight of maleic anhydride
(c) 0 to 49% by weight of additional (meth)acrylate monomer chosen from:

(i) linear or branched, saturated or unsaturated $C_1$-$C_{20}$ alkyl (meth)acrylates, optionally interrupted with one or more non-adjacent heteroatoms chosen from O and S or with a group NR, R being a $C_1$-$C_4$ alkyl group, optionally substituted with a phenyl or furfuryl group;
(ii) saturated $C_4$-$C_8$ cycloalkyl (meth)acrylates optionally interrupted with O or NH;

said amino alkoxysilane having the formula (I):

$$R\text{-}NH\text{-}R_1Si(OR_2)_z(R_3)_x \qquad (I)$$

in which:

$R_1$ is a linear or branched, saturated or unsaturated, cyclic or acyclic $C_1$-$C_{20}$ hydrocarbon-based divalent group, which may be interrupted in its chain with a heteroatom (O, S, NH) or a carbonyl group (CO), $R_1$ being linked to the silicon atom directly via a carbon atom;
R = H or a $C_1$-$C_4$ alkyl group, preferably H;
$R_2$ and $R_3$, which may be identical or different, represent a linear or branched alkyl group comprising from 1 to 6 carbon atoms,
z denotes an integer ranging from 1 to 3, and
x denotes an integer ranging from 0 to 2,
with z + x = 3.

**2.** Process according to Claim 1, **characterized in that** said maleic anhydride acrylic polymer is derived from the polymerization of:

(a) 50% to 80% by weight, relative to the total weight of monomers, of isobornyl (meth)acrylate
(b) 5% to 30% by weight of maleic anhydride
(c) 15% to 30% by weight of said additional (meth)acrylate monomer.

**3.** Process according to either of the preceding claims, **characterized in that** said maleic anhydride acrylic polymer is derived from the polymerization of:

(a) 60% to 80% by weight, relative to the total weight of monomers, of isobornyl (meth)acrylate
(b) 5% to 25% by weight of maleic anhydride
(c) 15% to 30% by weight of said additional (meth)acrylate monomer.

**4.** Process according to one of the preceding claims, **characterized in that** the maleic anhydride acrylic polymer is derived from the polymerization of:

(a) 50% to 80% by weight, relative to the total weight of monomers, of isobornyl (meth)acrylate
(b) 5% to 15% by weight of maleic anhydride
(c) 15% to 30% by weight of said additional (meth)acrylate monomer.

**5.** Process according to one of the preceding claims, **characterized in that** the maleic anhydride acrylic polymer is derived from the polymerization of:

(a) 60% to 80% by weight, relative to the total weight of monomers, of isobornyl (meth)acrylate
(b) 5% to 12% by weight of maleic anhydride
(c) 18% to 30% by weight of said additional (meth)acrylate monomer.

**6.** Process according to one of the preceding claims, **characterized in that** said additional (meth)acrylate monomer is chosen from $C_6$-$C_{16}$ alkyl (meth) acrylates and preferably from $C_6$-$C_{16}$ alkyl acrylates.

**7.** Process according to one of the preceding claims, **characterized in that** said maleic anhydride acrylic polymer is derived from the polymerization of:

(a) 50% to 80% by weight, relative to the total weight of monomers, of isobornyl (meth)acrylate
(b) 5% to 30% by weight of maleic anhydride
(c) 15% to 30% by weight of $C_6$-$C_{16}$ alkyl acrylate monomer.

**8.** Process according to one of the preceding claims, **characterized in that** the maleic anhydride acrylic polymer is derived from the polymerization of:

(a) 50% to 80% by weight, relative to the total weight of monomers, of isobornyl (meth)acrylate
(b) 5% to 30% by weight of maleic anhydride
(c) 15% to 30% by weight of $C_6$-$C_{16}$ alkyl acrylate monomer,

and **in that** it is preferably derived from the polymerization of:

(a) 50% to 80% by weight, relative to the total weight of monomers, of isobornyl (meth)acrylate
(b) 5% to 15% by weight of maleic anhydride
(c) 15% to 30% by weight of $C_6$-$C_{16}$ alkyl acrylate monomer.

**9.** Process according to one of the preceding claims, **characterized in that** the acrylic polymer comprises isobornyl acrylate, 2-ethylhexyl acrylate and maleic anhydride.

**10.** Process according to any one of the preceding claims, **characterized in that** the acrylic polymer has a weight-average molecular weight ranging from 5000 to 1 000 000 g/mol, preferably ranging from 10 000 to 500 000 g/mol, preferentially ranging from 15 000 to 350 000 g/mol.

**11.** Process according to any one of the preceding claims, **characterized in that**, for the amino alkoxysilane (I) used in the process according to the invention:

$$R = H;$$

$R_1$ is a linear saturated $C_1$-$C_6$ and preferably $C_2$-$C_4$ hydrocarbon-based chain;

$R_2$ represents an alkyl group, preferably a linear alkyl group, comprising from 1 to 4 carbon atoms, and preferably represents an ethyl group;
$R_3$ represents an alkyl group, preferably a linear alkyl group, comprising from 1 to 4 carbon atoms, and preferably represents a methyl or ethyl group;

preferably:

$R_1$ is a substituted linear saturated $C_1$-$C_6$ hydrocarbon-based chain,

$$R = H,$$

$R_2$ represents an alkyl group comprising from 1 to 4 carbon atoms,
$R_3$ represents an alkyl group comprising from 1 to 4 carbon atoms.

**12.** Process according to any one of the preceding claims, **characterized in that** the amino alkoxysilane (I) is chosen from 3-aminopropyltriethoxysilane, 3-aminoethyltriethoxysilane, 3-aminopropylmethyldiethoxysilane, N-(2-aminoethyl)-3-aminopropyltriethoxysilane, 3-(m-aminophenoxy)propyltrimethoxysilane, p-aminophenyltrimethoxysilane and N-(2-aminoethylaminomethyl)phenethyltrimethoxysilane; preferably chosen from 3-aminopropyltriethoxysilane (APTES), 3-aminoethyltriethoxysilane (AETES), 3-aminopropylmethyldiethoxysilane and N-(2-aminoethyl)-3-aminopropyltriethoxysilane; preferentially, it is 3-aminopropyltriethoxysilane.

**13.** Process according to any one of the preceding claims, **characterized in that** the amino alkoxysilane (I) is used in a mole ratio of amino alkoxysilane/maleic anhydride group of the acrylic polymer ranging from 0.01 to 10, preferably ranging from 0.1 to 5, preferentially ranging from 0.1 to 2 and more preferentially ranging from 0.1 to 1.

**14.** Process according to any one of the preceding claims, **characterized in that** the acrylic polymer is present in a content ranging from 0.1% to 10% by weight, relative to the total weight of the composition, preferably from 0.5% to 10% by weight, preferentially ranging from 1% to 8% by weight, and more preferentially ranging from 1% to 6% by weight.

**15.** Process according to any one of the preceding claims, **characterized in that** the composition comprises a hydrocarbon-based oil, preferably an apolar hydrocarbon-based oil containing from 8 to 14 carbon atoms, preferentially isododecane.

**16.** Process according to any one of the preceding claims, **characterized in that** a mixture, prepared less than 5 minutes before application to keratin materials, of the composition comprising the maleic anhydride acrylic polymer and of the amino alkoxysilane or of the composition containing same is applied to the keratin materials.

**17.** Process according to any one of claims 1 to 15, **characterized in that** the composition comprising the maleic anhydride acrylic polymer is first applied to keratin materials, and the amino alkoxysilane (I) or an anhydrous composition containing same and comprising a physiologically acceptable medium is then applied.

**18.** Process according to any one of claims 1 to 15, **characterized in that** the amino alkoxysilane (I), or an anhydrous composition containing same and comprising a physiologically acceptable medium, is first applied to keratin materials, and the composition comprising the maleic anhydride acrylic polymer is then applied.

**19.** Process according to any one of the preceding claims, **characterized in that** it is applied to the skin, more particularly the facial skin, in particular wrinkled skin.

**20.** Process according to any one of the preceding claims, **characterized in that** it is intended for attenuating wrinkles.

**21.** Cosmetic use, as a tensioning agent for the skin, in particular for wrinkled skin, of a maleic anhydride acrylic polymer as defined in any one of claims 1 to 10 and as a mixture with an amino alkoxysilane (I), or of an anhydrous composition containing same and comprising a physiologically acceptable medium, as defined in one of claims 1, 11 and 12.

**22.** Composition obtained by mixing an anhydrous composition comprising a maleic anhydride acrylic polymer as defined in any one of claims 1 to 10 and an amino alkoxysilane (I) or an anhydrous composition containing same and

comprising a physiologically acceptable medium, as defined in one of claims 1, 11 and 12.

**23.** Composition according to the preceding claim, **characterized in that** it is a makeup composition comprising a volatile oil and a nonvolatile oil, preferably a hydrocarbon-based volatile oil and a hydrocarbon-based nonvolatile oil.

**24.** Kit comprising a first anhydrous composition comprising a maleic anhydride acrylic polymer as defined in any one of claims 1 to 10 and a second anhydrous composition comprising an amino alkoxysilane (I) as defined in one of claims 1, 11 and 12 and comprising a physiologically acceptable medium, the first and second compositions each being packaged in a separate packaging assembly.

**25.** Polymer that may be obtained by reacting an amino alkoxysilane (I) as defined in one of claims 1, 11 and 12 with a maleic anhydride acrylic polymer as defined in any one of claims 1 to 10.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2008155059 A